**Europäisches Patentamt**

**European Patent Office**

**Office européen des brevets**

(19)

(11) Veröffentlichungsnummer: **0 337 273 B1**

(12) **EUROPÄISCHE PATENTSCHRIFT**

(45) Veröffentlichungstag der Patentschrift: **22.09.93**

(21) Anmeldenummer: **89105945.3**

(22) Anmeldetag: **05.04.89**

(51) Int. Cl.$^5$: **C07C 2/70**

(54) Verfahren zur Ethylierung oder Propylierung von Benzol.

(30) Priorität: **15.04.88 DE 3812516**
**25.08.88 DE 3828831**

(43) Veröffentlichungstag der Anmeldung:
**18.10.89 Patentblatt 89/42**

(45) Bekanntmachung des Hinweises auf die
Patenterteilung:
**22.09.93 Patentblatt 93/38**

(84) Benannte Vertragsstaaten:
**BE DE ES FR GB IT NL**

(56) Entgegenhaltungen:
**FR-A- 2 330 663**
**US-A- 2 394 905**

(73) Patentinhaber: **Linde Aktiengesellschaft**
**Abraham-Lincoln-Strasse 21**
**D-65189 Wiesbaden(DE)**

(72) Erfinder: **Zimmermann, Heinz, Dr.-Ing.**
**Irminsulstrasse 14a**
**D-8000 München 71(DE)**
Erfinder: **Häussinger, Peter, Dr. rer. nat.**
**Am Brombeerschlag 26**
**D-8000 München 70(DE)**

(74) Vertreter: **Schaefer, Gerhard, Dr.**
**Linde Aktiengesellschaft Zentrale Patentab-**
**teilung**
**D-82049 Höllriegelskreuth (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 337 273 B1

**Beschreibung**

Die Erfindung betrifft ein Verfahren zur Ethylierung oder Propylierung von Benzol mit Olefinen an einem festen Katalysator, der auf einem Träger aus $Al_2O_3$ Fluorverbindungen der Elemente der IV. und/oder V.Hauptgruppe des Periodensystems enthält.

Als besonders wichtige Alkylaromaten haben sich Ethylbenzol und das als Cumol bekannte i-Propylbenzol erwiesen. Ethylbenzol hat als Vorprodukt des wichtigen Kohlenwasserstoffes Styrol eine große Bedeutung erlangt. Cumol wird in erster Linie zur Synthese von Phenol und Aceton benutzt.

Ebenfalls von Bedeutung ist die Alkylierungsreaktion bei der Herstellung von Kraftstoff.

Für die Herstellung von Benzin wird Rohöl zunächst in eine Rohbenzinfraktion und eine Fraktion schwerer Kohlenwasserstoffe separiert. Nach einer Reformierung der Rohbenzinfraktion entsteht eine aromatenreiche, insbesondere Benzol, Toluol und Xylol enthaltende Kohlenwasserstofffraktion. Dabei ist ein möglichst hoher Gesamtaromatengehalt erwünscht, da er ausschlaggebend für die Oktanzahl des Kraftstoffes ist. Zur Verringerung des Benzolgehaltes wird diese Fraktion anschließend noch einer Alkylierung mit Olefinen unterzogen.

Die bei der Rohölauftrennung gewonnene Fraktion schwerer Kohlenwasserstoffe wird zur Herstellung von Benzin ebenfalls raffiniert. Dies kann auf verschiedene Arten, beispielsweise durch Fluidized-Catalytic-Cracking (FCC), Hydrocracking oder Thermocracking geschehen. Wird diese Fraktion mittels FCC weiterbehandelt, fällt eine flüssige Benzinfraktion und ein Ethylen, Ethan und Propan enthaltendes Abgas an. Dies wird zumeist in Raffinerien ohne Petrochemie-Anschluß hydriert und als Heizgas verwendet.

Im Prinzip haben sich zwei Verfahren durchgesetzt, nämlich die Flüssigphasen- und Gasphasenalkylierung, welche z.B. in "Industrielle organische Chemie", K. Weissermel, H.J. Arpe, 2. Auflage, 1978, Verlag Chemie, Seiten 315 bis 324 beschrieben sind.

Dabei erfolgt die Herstellung von Ethyl- und Propylbenzol durch Alkylierung von Benzol mit Ethylen bzw. Propylen.

Die Flüssigphasenalkylierung wird im allgemeinen unter Einsatz von Friedel-Crafts-Katalysatoren, wie z.B. $AlCl_3$, durchgeführt. Grundsätzlich sind die Katalysatoren sowohl für die Herstellung von Ethyl- als auch Propylbenzol gleichermaßen wirksam. Die Reaktionen werden - jeweils in Abhängigkeit vom vorgegebenen Druck oder der vorgegebenen Temperatur - bei Temperaturen zwischen 35 und 95 °C und Drücken bis 7 bar durchgeführt.

Nachteilig ist aber bei der Flüssigphasenalkylierung, daß die verwendeten Katalysatoren stark korrosiv wirken. Daher müssen zahlreiche Teile der Anlage korrosionsfest ausgestattet sein, d.h. die Reaktoren müssen beispielsweise innen mit säurefesten Keramikziegeln ausgemauert sein, ebenso müssen die Rohrleitungen, welche mit dem Reaktionsprodukt in Berührung kommen, aus säurefestem und damit teurem Material gefertigt sein.

Außerdem liegt der Katalysator nach der Flüssigphasenalkylierung in suspendierter bzw. gelöster Form vor, womit eine nachfolgende Abtrennung durch Wäschen sowie daran anschließend eine Trocknung rückgeführten Benzols notwendig ist, was einen erheblichen apparativen Aufwand sowie hohe Betriebskosten bedeutet.

Demgegenüber steht als zweites Verfahren die Gasphasenalkylierung von Benzol oder aromatenreichen Kraftstofffraktionen mit Ethylen bzw. Propylen an Zeolithen oder sauren Trägerkatalysatoren wie z.B. $H_3PO_4/SiO_2$ oder $BF_3/Al_2O_3$. Je nach verwendetem Katalysator und Druck/Temperatur-Verhältnis finden die Reaktionen bei Temperaturen im Bereich von 250 bis 450 °C und Drücken im Bereich von 3 bis 65 bar statt, wobei allgemein die Alkylierung zu Ethylbenzol bei höheren Drücken und Temperaturen als die Alkylierung zu Propylbenzol erfolgt.

Außerdem wirken viele der sauren Trägerkatalysatoren ebenfalls korrosiv und sind nicht regenerierbar wie beispielsweise $H_3PO_4/SiO_2$, was wiederum negative Auswirkungen auf Investitions- und Betriebskosten hat und eine aufwendige Entsorgung des verbrauchten Katalysators erfordert.

Aus der FR-PA 2 330 663 ist eine Verfahren zur Umwandlung von Kohlenwasserstoffen, bei dem man unter Ausbildung einer Kohlenwasserstoffphase und einer Katalysatorphase ein Kohlenwasserstoffinsatzmaterial mit einem Katalysator in Kontakt bringt, bekannt. Hierbei wird ein Katalysator verwendet, der aus mindestens einer Lewis-Säure und einer starken Brönsted-Säure besteht. Ferner wird bei einer teilweisen Desaktivierung der Katalysatorphase der Katalysator regeneriert, indem er in einer Regenerierungszone bei einer Temperatur von 40 bis 500 °C mit Wasserstoff in Kontakt gebracht wird.

Der vorliegenden Erfindung liegt die Aufgabe zugrunde, ein Verfahren der eingangs genannten Art zur Verfügung zu stellen, mit dem auf einfache und kostengünstige Weise hohe Ausbeuten an Alkylaromaten erzielt werden können.

2

Diese Aufgabe wird dadurch gelöst, der Katalysator aus einem $Al_2O_3$-Träger mit 0,1 bis 30 Gew.%, vorzugsweise 5 bis 15 Gew.%, supersaurem $H_2SiF_6$ oder $HPF_6$ besteht.

Erfindungsgemäß werden die Supersäuren $H_2SiF_6$ oder $HPF_6$ verwendet.

Der erfindungsgemäß für die Ethylierung oder Propylierung eingesetzte Katalysator wird in an sich bekannter Weise durch Tränken des Trägers mit den Supersäuren $H_2SiF_6$ oder $HPF_6$ oder deren Salzlösungen und nachfolgender Calcinierung hergestellt, wobei die supersaure Komponente chemisch auf dem Träger gebunden wird.

Aufgrund der Trägerfixierung der Supersäure sind die Reaktionsmedien nicht korrosiv, was zusammen mit milden Reaktionsbedingungen zu günstigen Investitionskosten führt.

Die erfindungsgemäßen Supersäuren sind stärker sauer als eine 100%ige Schwefelsäure. Sie besitzen in der Regel einen pH-Wert von etwa -0,5.

Mit einem erfindungsgemäßen supersauren Katalysator können hervorragende Ausbeuten bei hoher Selektivität und milden Reaktionsbedingungen erzielt werden. Insbesondere kann die Ethylierung oder Propylierung bei einem Druck von 3 bis 30 bar, vorzugsweisen 3 bis 7 bar, bei einer Temperatur zwischen 200 bis 500°C, vorzugsweise 300 bis 400°C und einer Raumgeschwindigkeit GHSV "gaseous hourly space velocity") zwischen 300 und 920 l/h durchgeführt werden.

Aufgrund der hohen Acidität des Katalysators kann insbesondere die Ethylierung von Benzol bei einem Druck von 5 bar, einer Temperatur von 400°C, einer Raumgeschwindigkeit GHSV von 780 l/h und einem Molverhältnis Benzol/Ethylen von 5 durchgeführt werden. Die Propylierung von Benzol kann bei einem Druck von 5 bar, einer Temperatur von 300°C, einer Raumgeschwindigkeit GHSV von 460 l/h und einem Molverhältnis Benzol/Propylen von 5 durchgeführt werden. Der eingesetzte Aromat, in erster Linie Benzol, sollte keine Verunreinigungen mit anderen aromatischen Kohlenwasserstoffen, wie Toluol, Xylol enthalten. Diese würden zur Bildung unerwünschter Nebenprodukte führen, die nicht zu den Zielprodukten umalkyliert werden können und damit die Ausbeute verringern.

Für die Alkylierung ist es dabei nicht erforderlich, reines Ethylen/Propylen zu verwenden. Vielmehr können auch $C_2$- bzw. $C_3$-Schnitte eingesetzt werden. Das in derartigen Schnitten enthaltene Ethan bzw. Propan ist inert und stört somit die Reaktion nicht. Es ist lediglich darauf zu achten, daß Ethylen bzw. Proplyen in ausreichender Menge im Einsatz vorhanden sind.

Es wird mit Vorteil immer mit einem Aromatenüberschuß gearbeitet, der zwischen 2 und 11, vorzugsweise zwischen 4 und 6 in Bezug auf Ethylen bzw. Propylen betragen sollte. Auf diese Weise wird eine möglichst große Ausbeute an Monoethyl- bzw. Monopropylbenzol erhalten, wobei Ethylen bzw. Propylen praktisch quantitativ umgesetzt werden.

Es findet aber dennoch stets eine Weiteralkylierung statt, da die Geschwindigkeitskonstanten der einzelnen Alkylierungsstufen sehr ähnlich sind, wobei bei der Ethylierung von Benzol in erster Linie Di- und Tripropylbenzol, bei der Propylierung von Benzol Di- und Tripropylbenzol gebildet werden. Diese Nebenprodukte können gemäß einer vorteilhaften Weiterbildung des erfindungsgemäßen Verfahrens vom Alkylierungsprodukt in an sich bekannter Weise, z.B. destillativ, abgetrennt und zur Alkylierung zurückgeführt werden. Dort werden die Nebenprodukte sodann während der Alkylierung unter den Alkylierbedingungen beispielsweise gemäß

Diethylenbenzol + Benzol ----> 2 Ethylbenzol

umalkyliert.

Bisher war es üblich, die Umalkylierung der Nebenprodukte aus einer Gasphasenalkylierung entweder am gleichen Katalysator, jedoch in einem getrennten Umalkylierungsreaktor bei anderen als den Alkylierungsbedingungen durchzuführen, beispielsweise bei höheren Temperaturen, oder aber die Alkylierungskatalysatoren wie z.B. $H_3PO_4/SiO_2$ sind grundsätzlich nicht für die Umalkylierung aktiv.

Mit dem erfindungsgemäßen umalkylieraktiven Katalysator jedoch können Alkylierung und Umalkylierung gleichzeitig in einem einzigen Reaktor stattfinden. Damit können die Ausbeuten an Mono-Alkylierungsptodukten erheblich gesteigert werden.

Die Alkylierungsreaktion läuft stark exotherm auf einem günstigen Temperaturniveau ab, so daß Hochdruckdampf gewonnen werden kann. Als besonders günstig hat es sich dabei erwiesen, wenn die bei der Alkylierung freiwerdende Energie bei einer nachfolgenden Dehydrierung des Alkylierungsprodukts genutzt wird. So kann beispielsweise die exotherme Alkylierung zu Ethylbenzol mit der endothermen Dehydrierung des Ethylbenzols zu Styrol in energiesparender Weise kombiniert werden.

Gemäß einer weiteren vorteilhaften Ausgestaltungsform des erfindungsgemäßen Verfahrens wird die Ethylierung oder Propylierung von Benzol aus einer aromatenreichen Kraftstofffraktion und dem Abgas einer Fluidized-Catalytic-Cracking Anlage durchgeführt.

3

Damit wird erfindungsgemäß vorgeschlagen, das sonst meist zu Heizzwecken verwendete Abgas der FCC-Stufe im Prozeß zur Verringerung des Benzolgehaltes einzusetzen. Eine aromatenreiche Kraftstofffraktion sowie das FCC-Abgas werden erfindungsgemäß einer Gasphasenalkylierung zugeführt. Die im Abgas neben Ethylen enthaltenen Komponenten Ethan und Propan verhalten sich bei der Umsetzung inert und stören daher nicht.

Das in dieser Alkylierung gebildete Ethyl- bzw. Diethylbenzol verbleibt in der umgesetzten Kraftstofffraktion, die von den nicht umgesetzten Gasprodukten abgetrennt wird. Die so abgetrennte Gasphase, überwiegend Ethan und Propan enthaltend, kann als Heizgas genutzt werden.

Neben Benzol werden in geringerem Ausmaß die weiteren in der Kraftstofffraktion enthaltenen Aromaten alkyliert. Dies ist jedoch nicht von Nachteil, da der Gesamtaromatenanteil nicht abnimmt, welcher für die Oktanzahl des Kraftstoffes ausschlaggebend ist. Eine Entfernung der von Benzol verschiedenen Aromaten vor der Alkylierung ist daher nicht nötig.

Es sollte auch bei der Alkylierung der aromatenreichen Kraftstofffraktion immer mit einem Aromatenüberschuß gearbeitet werden, der zwischen 2 und 11, vorzugsweise zwischen 4 und 6 in Bezug auf Ethylen betragen sollte. Auf diese Weise wird ein möglichst großer Umsatz zu Ethylbenzol bzw. Diethylbenzol erhalten, wobei Ethylen praktisch quantitativ umgesetzt wird.

Das erfindungsgemäße Verfahren bietet weiterhin den Vorteil, daß der verwendete Katalysator leicht regenerierbar ist, d.h. die sich während der Reaktion bildenden Koks- und Polymerablagerungen sich auf einfache und billige Weise mit angereicherter Luft wieder entfernen lassen.

Im folgenden sei die Erfindung anhand zweier schematisch dargestellter Ausführungsbeispiele näher erläutert.

Figur 1    Alkylierung von Benzol mit Ethylen

Figur 2    Alkylierung einer aromatenreichen Kraftstofffraktion mit dem Abgas einer FCC-Stufe

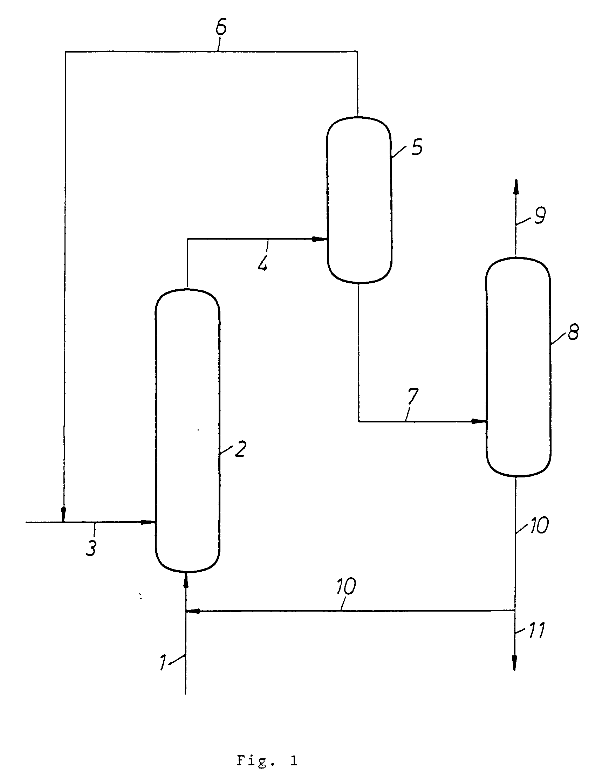

Gemäß Figur 1 wird über Leitung 1 Benzol einem Reaktor 2 zugeführt. In diesem findet in Gegenwart von über Leitung 3 herangeführtem Ethylen über einem Katalysator bestehend aus einem $Al_2O_3$-Träger mit 10 Gew.% $H_2SiF_6$ die Alkylierung zu Ethylbenzol statt. Der Reaktor steht dabei unter einem Druck von 5 bar.

Über Leitung 4 wird das Alkylierungsprodukt einem Flash-Verdampfer 5 zugeführt, in dem eine Auftrennung in eine gasförmige und eine flüssige Phase stattfindet. Die Gasphase, die überwiegend aus Ethylen besteht, wird über Leitung 6 abgezogen und dem Einsatz Ethylen in Leitung 3 zugemischt. Die Flüssigphase, die aus Ethylbenzol und Nebenprodukten, in erster Linie Di- und Triethylbenzol besteht, wird über Leitung 7 einer Destillationskolonne 8 zugeführt und destillativ aufgetrennt. Über Kopf der Kolonne 8 kann über Leitung 9 das Ethylbenzolprodukt gewonnen werden. Über Leitung 10 werden die Nebenprodukte abgezogen und zum Reaktor 2 zur Umalkylierung zurückgeführt. Je nach den Erfordernissen besteht auch die Möglichkeit, diese Nebenprodukte über eine Leitung 11 ohne Umalkylierung abzuziehen.

Anstelle des Flash-Verdampfers kann auch eine Strippsäule verwendet werden, in der beispielsweise mittels Ethylen als Strippgas das Gas ausgestrippt wird.

In der nachfolgenden Tabelle 1 sind Versuchsergebnisse dargestellt. Der Druck beträgt für alle Versuche 5 bar. In Tabelle 1 wurden folgende Definitionen verwendet:

GHSV    N1 (Benzol + Ethylen) gasförmig/l (Katalysator)h

B/E        Benzol/Ethylenverhältnis (mol/mol)

EB         Ethylbenzol

DEB       Diethylbenzol

UE         Umsatz Ethylen

SEB        Selektivität Ethylbenzol.

Tabelle 1

| GHSV h⁻¹ | B/E mol/mol | T_max °C | EB mol% | DEB mol% | UE % | SEB % |
|---|---|---|---|---|---|---|
| 459 | 6,46 | 341 | 8,46 | 0,26 | 58,04 | 97,01 |
| 491 | 4,28 | 347 | 10,83 | 0,496 | 50,62 | 91,60 |
| 491 | 4,28 | 462 | 22,02 | 0,56 | 99,01 | 97,50 |
| 521 | 3,23 | 477 | 23,5 | 2,50 | 92,02 | 90,38 |
| 669 | 8,21 | 489 | 11,74 | 0,10 | 98,17 | 99,12 |
| 713 | 5,86 | 461 | 14,61 | 0,93 | 96,52 | 94,02 |
| 674 | 8,27 | 393 | 10,19 | 0,34 | 89,58 | 96,81 |
| 652 | 5,27 | 423 | 16,28 | 0,94 | 95,68 | 94,56 |
| 774 | 4,71 | 418 | 17,24 | 1,20 | 92,59 | 93,49 |
| 509 | 10,98 | 302 | 5,38 | 0,335 | 66,87 | 93,80 |
| 623 | 4,98 | 410 | 15,48 | 1,073 | 87,95 | 93,5 |
| 516 | 3,96 | 414 | 17,88 | 2,095 | 87,54 | 89,11 |
| 546 | 3,03 | 432 | 22,01 | 3,171 | 85,91 | 87,40 |
| 495 | 10,64 | 350 | 7,26 | 0,17 | 80,93 | 97,71 |
| 529 | 6,28 | 387 | 11,91 | 0,65 | 82,97 | 94,82 |
| 574 | 4,52 | 406 | 15,83 | 1,438 | 84,55 | 91,66 |
| 612 | 3,517 | 415 | 17,54 | 2,54 | 79,62 | 87,31 |
| 724 | 8,97 | 380 | 8,73 | 0,099 | 80,14 | 98,87 |
| 780 | 6,50 | 403 | 11,64 | 0,48 | 81,97 | 96,03 |
| 831 | 5,13 | 415 | 13,93 | 0,825 | 79,93 | 94,40 |
| 858 | 4,109 | 430 | 16,94 | 1,258 | 79,96 | 93,08 |
| 887 | 3,47 | 440 | 19,06 | 1,606 | 77,44 | 92,22 |
| 885 | 11,18 | 375 | 6,54 | 0,286 | 79,63 | 95,81 |
| 918 | 7,831 | 389 | 9,16 | 0,518 | 74,37 | 94,64 |

Wie aus Tabelle 1 klar wird, sind die Umsätze stark von der Raumgeschwindigkeit GHSV, dem Verhältnis B/E und der Temperatur abhängig.

Für die Prüfung des Katalysators auf Umalkylierung wurde zunächst Benzol mit Ethylen, danach eine Mischung Benzol/Ethylenbenzol/Diethylbenzolmit Ethylen und abschließend wiederum Benzol mit Ethylen alkyliert. Während dieses Versuches wurden alle Parameter (GHSV, B/E, Druck) konstant gehalten. Die Ergebnisse des Versuches sind in Tabelle 2 gezeigt.

## Tabelle 2
### Nachweis der Umalkylierung

| Zeit (min) | Einsatz | % Ethyl-benzol | % DEB | T |
|---|---|---|---|---|
| 36 | Benzol | 14,39 | 1,483 | 412 |
| 150 | " | 14,65 | 1,349 | |
| 196 | Benzol 89,62 %<br>Ethylbenzol 3,69 %<br>Diethylbenzol 5,95 % | | | |
| 248 | " | 25,40 | 3,64 | 452 |
| 256 | Benzol | | | |
| 340 | | 16,03 | 1,26 | 448 |
| 376 | | 16,44 | 1,29 | 452 |
| 398 | | 16,59 | 1,27 | 450 |

B/E = const.    P = const.

GHSV = const.

Umsatz bei der Umalkylierung:

$$\frac{\text{Produkt}}{-1,27 \cdot 0,89} = \frac{3,64 \text{ DEB}}{-1,13 \text{ DEB (aus Benzol + Ethylen)}}$$

$$2,51 \text{ DEB (durch Umalkylierung)}$$

$$U_{DEB} = 100 - \frac{100 \cdot 2,51}{5,95} = 57,8 \text{ %}$$

Aus Tabelle 2 ist zu erkennen, daß der Katalysator umalkylieraktiv ist, wobei ca. 60% des eingesetzten Diethylbenzols in Ethylbenzol umgewandelt werden.

Neben der Ethylbenzolherstellung aus Benzol und Ethylen ist die Cumolherstellung aus Benzol und Propylen von Interesse. Auch hierzu wurden Vrsuche bei konstantem Druck von 5 bar durchgeführt. Die Versuchsergebnisse sind in Tabelle 3 zusammengestellt.

6

Tabelle 3

| GHSV $h^{-1}$ | B/P mol/mol | $T_{max}$ °C | Cumol mol% | Alkylbenzol mol% | $U_{Propylen}$ % | S Cumol % |
|---|---|---|---|---|---|---|
| 451 | 7,44 | 300 | 5,82 | 0,84 | 49,70 | 87,38 |
| 460 | 5,70 | 300 | 11,32 | 0,24 | 66,02 | 97,92 |
| 490 | 4,93 | 304 | 16,71 | 0,64 | 83,53 | 96,22 |
| 521 | 3,75 | 308 | 19,59 | 0,69 | 76,15 | 96,59 |

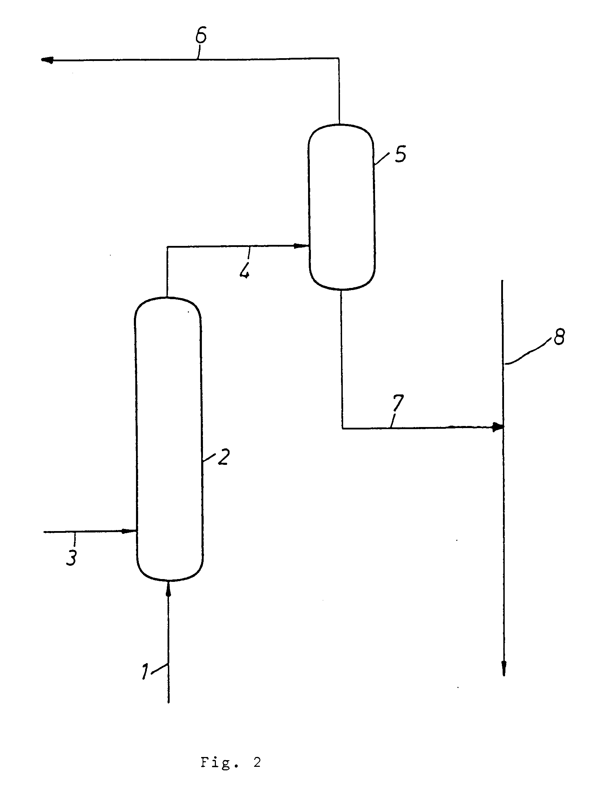

Gemäß Figur 2 wird über Leitung 1 eine Aromaten, darunter Benzol, enthaltende Kraftstofffraktion einem Reaktor 2 zugeführt In diesem findet in Gegenwart von über Leitung 3 herangeführtem ethylenhaltigem Abgas einer FCC-Stufe die Alkylierung zu Ethylbenzol bzw. Diethylbenzol statt, unter Einsatz eines $H_2SiF_6$-Katalysators mit $Al_2O_3$ als Trägermaterial. Über Leitung 4 wird das Alkylat einem Abscheider 5 zugeführt, in welchem eine Abtrennung in eine gasförmige und eine flüssige Phase stattfindet. Diese Gasphase, hauptsächlich aus Ethan und Propan bestehend, wird über Leitung 6 abgezogen und kann als Heizgas verwendet werden. Die Flüssigphase, die aus einem Kraftstoffgemisch mit Ethyl bzw. Diethylbenzol und anderen aromatischen Nebenprodukten besteht, wird mittels Leitung 7 abgezogen und über Leitung 8 dem Benzinpool zugeführt.

## Patentansprüche

1. Verfahren zur Ethylierung oder Propylierung von Benzol mit Olefinen an einem festen Katalysator, der auf einem Träger aus $Al_2O_3$ Fluorverbindungen der Elemente der IV. und/oder V.Hauptgruppe des Periodensystems enthält, **dadurch gekennzeichnet,** daß der Katalysator aus einem $Al_2O_3$-Träger mit 0,1 bis 30 Gew.%, vorzugsweise 5 bis 15 Gew.%, supersaurem $H_2SiF_6$ oder $HPF_6$ besteht.

2. Verfahren nach Anspruch 1, dadurch gekennzeichnet, daß die Ethylierung oder Propylierung bei einem Druck von 3 bis 30 bar, vorzugsweise 3 bis 7 bar, einer Temperatur von 200 bis 500°C, vorzugsweise 300 bis 400°C und einer Raumgeschwindigkeit GHSV ("gaseous hourly space velocity") von 300 bis 920 l/h durchgeführt wird.

3. Verfahren nach Anspruch 1 oder 2, dadurch gekennzeichnet, daß die Ethylierung von Benzol bei einem Druck von 5 bar, einer Temperatur von 400°C, einer Raumgeschwindigkeit GHSV von 780 l/h und einem Molverhältnis Benzol/Ethylen von 5 durchgeführt wird.

4. Verfahren nach Anspruch 1 oder 2, dadurch gekennzeichnet, daß die Propylierung von Benzol bei einem Druck von 5 bar, einer Temperatur von 300°C, einer Raumgeschwindigkeit GHSV von 460 l/h und einem Molverhältnis Benzol/Propylen von 5 durchgeführt wird.

5. Verfahren nach einem der Ansprüche 1 bis 4, dadurch gekennzeichnet, daß die bei der Ethylierung oder Propylierung entstehenden Nebenprodukte vom Ethylier- bzw. Propylierprodukt abgetrennt, zur Ethylierung oder Propylierung zurückgeführt und umethyliert bzw. umpropyliert werden.

6. Verfahren nach einem der Ansprüche 1 bis 5, dadurch gekennzeichnet, daß bei der Ethylierung oder Propylierung freiwerdende Energie bei einer nachfolgenden Dehydrierung des Ethylier- bzw. Propylierprodukts genutzt wird.

7. Verfahren nach einem der Ansprüche 1 bis 6, dadurch gekennzeichnet, daß die Ethylierung oder Propylierung von Benzol aus einer aromatenreichen Kraftstofffraktion und dem Abgas einer Fluidized-Catalytic-Cracking-Anlage durchgeführt wird.

## Claims

1. A process for the ethylation or propylation of benzene with olefins in the presence of a static catalyst which, on a support composed of $Al_2O_3$, contains fluorine compounds of the elements of the IVth and/or Vth main groups of the periodic system, characterised in that the catalyst consists of an $Al_2O_3$ support containing 0.1 to 30 % by weight, preferably 5 to 15% by weight, super-acid $H_2SiF_6$ or $HPF_6$.

**2.** A process as claimed in Claim 1, characterised in that the ethylation or propylation is carried out at a pressure of 3 to 30 bar, preferably 3 to 7 bar, a temperature of 200 to 500 °C, preferably 300 to 400 °C and a GHSV (gaseous hourly space velocity) of 300 to 920 l/h.

**3.** A process as claimed in Claim 1 or 2, characterised in that the ethylation of benzene is carried out at a pressure of 5 bar, a temperature of 400 °C, a GHSV of 780 l/h and a molar ratio benzene/ethylene of 5.

**4.** A process as claimed in Claim 1 or 2, characterised in that the propylation of benzene is carried out at a pressure of 5 bar, a temperature of 300 °C, a GHSV of 460 l/h and a molar ratio benzene/propylene of 5.

**5.** A process as claimed in one of Claims 1 to 4, characterised in that the by-products produced by the ethylation or propylation are separated from the ethylation or propylation product respectively, recycled to the ethylation or propylation stage and re-ethylated or re-propylated respectively.

**6.** A process as claimed in one of Claims 1 to 5, characterised in that energy released in the ethylation or propylation is used in a subsequent dehydrogenation of the ethylation- or propylation product.

**7.** A process as claimed in one of Claims 1 to 6, characterised in that the ethylation or propylation of benzene is carried out on the basis of a fuel fraction high in aromatics and the exhaust gas from a fluidized-catalytic-cracking apparatus.

**Revendications**

**1.** Procédé d'éthylation ou de propylation de benzène avec des oléfines sur un catalyseur solide, qui contient sur un support à base d'$Al_2O_3$ des composés fluorés d'éléments des colonnes IV et/ou V du tableau de la classification périodique, caractérisé en ce que le catalyseur consiste en un support à base d'$Al_2O_3$ avec de 0,1 à 30%, de préférence 5 à 15% en poids, de superacide $H_2SIF_6$ ou $HPF_6$.

**2.** Procédé selon la revendication 1, caractérisé en ce que l'éthylation ou la propylation est mise en oeuvre sous une pression de 3 à 30 bars, de préférence de 3 à 7 bars, à une température de 200 à 500 °C, de préférence 300 à 400 °C, et avec une vitesse spatiale horaire à l'état gazeux VSHG de 300 à 920 litres/heure.

**3.** Procédé selon la revendication 1 ou 2, caractérisé en ce que l'éthylation de benzène est mise en oeuvre sous une pression de 5 bars, à une température de 400 °C, une vitesse spatiale horaire à l'état gazeux VSHG de 780 litres/heure et un rapport molaire benzène/éthylène de 5.

**4.** Procédé selon la revendication 1 ou 2, caractérisé en ce que la propylation de benzène est mise en oeuvre sous une pression de 5 bars, à une température de 300 °C, une vitesse spatiale horaire à l'état gazeux VSHG de 460 litres/heure et un rapport molaire benzène/propylène de 5.

**5.** Procédé selon l'une quelconque des revendications 1 à 4, caractérisé en ce que les sous-produits obtenus lors de l'éthylation ou de la propylation sont séparés du produit éthylé ou propylé, recyclés vers l'éthylation ou la propylation et transéthylés ou transpropylés.

**6.** Procédé selon l'une quelconque des revendications 1 à 5, caractérisé en ce que l'énergie libérée lors de l'éthylation ou de la propylation est utilisée au cours d'une déshydrogénation ultérieure du produit éthylé ou propylé.

**7.** Procédé selon l'une quelconque des revendications 1 à 6, caractérisé en ce que l'éthylation ou la propylation du benzène est mise en oeuvre à partir d'une fraction de carburant riche en aromatiques et de l'effluent gazeux d'une installation de craquage sur lit catalytique fluidisé.

Fig. 1

Fig. 2